# EUROPEAN PATENT APPLICATION

(11) **EP 4 458 437 A1**
(43) Date of publication of application: **06.11.2024**
(21) Application number: 22916753.1
(22) Date of filing: 28.12.2022
(51) Int. Cl.: A63B 24/00, A63B 71/06, G06N 3/08, G06N 20/00, G06V 10/778

(54) **AI EXERCISE GUIDE DEVICE AND METHOD**

(30) Priority: 28.12.2021 KR 20210190376; 14.11.2022 KR 20220152002
(71) Applicant: DRAX Inc., Anyang-si, Gyeonggi-do 14086 (KR)
(72) Inventor: YOO, Seon Kyung, Seoul 08251 (KR)
(74) Representative: Mewburn Ellis LLP
(86) International application number: PCT/KR2022/021513
(87) International publication number: WO 2023/128612

(57) **Abstract**

As a preferred embodiment of the present disclosure, an AI exercise guide device includes a muscle strength estimation unit for estimating an estimated muscle strength value of a user's specific muscle strength based on user data, a detection unit for detecting a percentile value to which the estimated muscle strength value belongs among preset adult muscle strength percentile values, and a PMW estimation unit for estimating a PMW of exercise equipment the user intends to use based on the detected percentile value.

## Description

### Technical Field

The present disclosure relates to a method and exercise equipment for automatically providing a suitable target weight to a user.

### Background Art

Weightlifting equipment is provided in various forms depending on the body part of which muscle strength is to be increased or the purpose of use, and is mainly used to train the upper body and the lower body using hands or feet. A user may perform an exercise by moving a selected weight through the structure of the weightlifting equipment.

However, it is difficult for the user to confirm whether he or she is using the exercise equipment appropriately according to his or her physical characteristics, exercise performance, or purpose of the exercise equipment.

### Disclosure

### Technical Problem

As a preferred embodiment of the present disclosure, an AI exercise guide device seeks to automatically set a target weight of exercise equipment a user intends to use when user data including the user's gender, age, weight, height, BMI, and body fat percentage is input.

As a preferred embodiment of the present disclosure, the AI exercise guide device seeks to automatically adjust the target weight of the exercise equipment according to the user's exercise purpose.

### Technical Solution

As a preferred embodiment of the present disclosure, an AI exercise guide device includes a muscle strength estimation unit for estimating an estimated muscle strength value of a user's specific muscle strength based on user data, a detection unit for detecting a percentile value to which the estimated muscle strength value belongs among preset adult muscle strength percentile values, and a PMW estimation unit for estimating a PMW of exercise equipment the user intends to use based on the detected percentile value.

### Advantageous Effects

As a preferred embodiment of the present disclosure, an AI exercise guide device has the effect of automatically setting a target weight of exercise equipment a user intends to use when user data including the user's gender, age, weight, height, BMI, and body fat percentage is input.

The AI exercise guide device also has the effect of automatically setting a target weight suitable for the user for each exercise equipment according to the user's exercise purpose.

### Description of Drawings

FIG. 1 is a diagram of a smart gym system where an AI exercise guide device is used, as a preferred embodiment of the present disclosure.
FIG. 2 is a diagram of an example of exercise equipment implemented with an AI exercise guide method in a smart gym, as a preferred embodiment of the present disclosure.
FIG. 3 is an internal configuration diagram of exercise equipment implemented with an AI exercise guide method and a smart gym server in a smart gym system, as a preferred embodiment of the present disclosure.
FIG. 4 is an internal configuration diagram of an AI exercise guide device, as a preferred embodiment of the present disclosure.
FIG. 5 is a diagram of an example of a user interface of an AI exercise guide device, as a preferred embodiment of the present disclosure.
FIG. 6 is a diagram of an example of exercise traces detected from exercise equipment implemented with an AI exercise guide method, as a preferred embodiment of the present disclosure.
FIG. 7 is a flowchart of an AI exercise guide method, as a preferred embodiment of the present disclosure.

### Best Mode

As a preferred embodiment of the present disclosure, an AI exercise guide device includes a muscle strength estimation unit for estimating an estimated muscle strength value of a user's specific muscle strength based on user data, a detection unit for detecting a percentile value to which the estimated muscle strength value belongs among preset adult muscle strength percentile values, and a PMW estimation unit for estimating a PMW of exercise equipment the user intends to use based on the detected percentile value.

As a preferred embodiment of the present disclosure, the AI exercise guide device further includes an exercise goal setting unit for automatically setting a target weight of exercise equipment the user intends to use based on the estimated PMW thereof.

As a preferred embodiment of the present disclosure, the PMW estimation unit estimates a PMW matching the estimated muscle strength value by using a matching table that matches a PMW percentile value of exercise equipment obtained from a population using the exercise equipment that the user intends to use with the preset adult muscle strength percentile value.

As a preferred embodiment of the present disclosure, the PMW percentile value of the exercise equipment obtained from the population is updated using PMW data of the user who used exercise equipment the user intended to use in at least one smart gym, and in this case, the matching table is updated.

As a preferred embodiment of the present disclosure, the user data includes at least some of a user's gender, age, weight, height, BMI, and body fat percentage.

As a preferred embodiment of the present disclosure, the specific muscle strength includes a grip strength.

As a preferred embodiment of the present disclosure, the target weight of the exercise equipment that the user intends to use varies depending on the user's exercise purpose received through a user interface.

As a preferred embodiment of the present disclosure, an AI exercise guide method includes calculating an estimated muscle strength value obtained by estimating a user's specific muscle strength based on user data by a muscle strength estimation unit, detecting a percentile value to which the estimated muscle strength value belongs among preset adult muscle strength percentile values by a detection unit, and estimating a PMW of exercise equipment the user intends to use based on a percentile value to which the estimated muscle strength value belongs by a PMW estimation unit.

### Mode for Invention

Hereinafter, some embodiments of the present disclosure are described in detail with reference to the attached drawings so that those skilled in the art can easily practice the present disclosure.

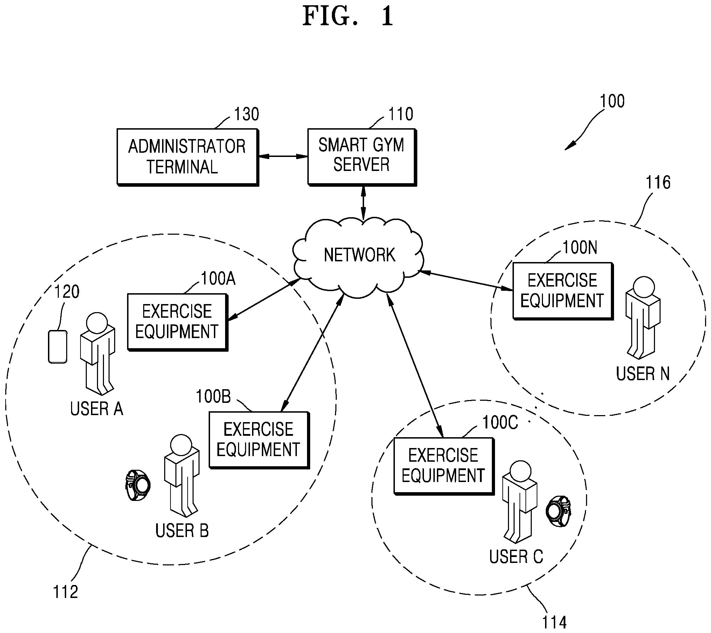

FIG. 1 is a diagram of a smart gym system where an AI exercise guide device is used, as a preferred embodiment of the present disclosure.

A smart gym system 100 includes a smart gym server 110, at least one piece of exercise equipment 100A, 100B, 100C, and 100N, at least one user terminal 120, and an administrator terminal 130.

In FIG. 1, the smart gym server 110 may communicate with a first smart gym 112, a second smart gym 114, and an nth smart gym 116 at physically different locations, and may exchange data with at least one piece of exercise equipment 100A and 100B in the first smart gym 112, at least one piece of exercise equipment 100C in the second smart gym 114, and at least one piece of exercise equipment 100N in the nth smart gym 116.

As a preferred embodiment of the present disclosure, a smart gym is a physical space where a user's record of using exercise equipment is provided to the smart gym server 110 and an exercise prescription tailored to the user is provided by the smart gym server 110 which learns and analyzes the user's record. The smart gym may be implemented as a fitness center, a gym, or a space equipped with exercise equipment.

Users USER A, USER B, USER C, and USER N who come to the smart gym to exercise may enter the smart gym after verifying their identity when entering the smart gym. For example, a user may enter the smart gym after the user's membership is confirmed by tagging the user terminal 120 to an unmanned terminal, such as a kiosk, at the smart gym entrance, using near field communication (NFC) or radio frequency identification (RFID) or may enter the smart gym after the user's membership is confirmed through biometric verification, such as facial recognition, on the unmanned terminal.

Information about the user whose membership has been confirmed may be transmitted from the smart gym server 110 to at least one piece of the exercise equipment 100A, 100B, 100C, and 100N through a network. For example, the smart gym server 110 may transmit the information about the user to the exercise equipment to which the user has tagged the terminal 120. In a preferred embodiment of the present disclosure, the information about the user is also referred to as user data and includes at least some or all of the user's gender, age, weight, height, BMI, and body fat percentage.

The smart gym server 110 may provide a first user USER A and a second user USER B who use at least one piece of the exercise equipment 100A and 100B in the smart gym 112, respectively, with an exercise method, an exercise intensity, and an exercise sequence, which are tailored to each user. Additionally, the smart gym server 110 may automatically control values, such as a target weight and a recommended usage speed of each exercise equipment 100A and 100B. Additionally, the smart gym server 110 may receive exercise records of the first user USER A and the second user USER B using the exercise equipment 100A and 100B, respectively. The smart gym server 110 may receive health information or life log information, such as a user's heart rate, blood pressure, pulse, etc., from the user terminal 120.

The smart gym server 110 may be implemented in the form of a cloud server. The smart gym server 110 may integrate and manage information collected from each exercise equipment in smart gyms located at different locations. For example, the smart gym server 110 may integrate and manage the details of the first user's use of the exercise equipment in the smart gym 112 at a first location and the details of the first user's use of the exercise equipment in the smart gym 114 at a second location.

As a preferred embodiment of the present disclosure, at least one piece of the exercise equipment 100A, 100B, 100C, and 100N may be stretching equipment, weightlifting equipment, or cardio equipment. The weightlifting equipment includes a free weight and a weight machine. At least one piece of the exercise equipment 100A, 100B, 100C, and 100N provides a suitable exercise guide to the user through a display attached to the exercise equipment or a display capable of wired or wireless communication with the exercise equipment. For example, the stretching equipment provides an exercise guide related to stretching for the user to use through a smart mirror capable of wired or wireless communication with the stretching equipment. However, it is not limited thereto, and the exercise guide may be provided using various output methods, such as speakers and vibration.

At least one piece of the exercise equipment 100A, 100B, 100C, and 100N may perform wired or wireless communication with the smart gym server 110, the user terminal 120, and the administrator terminal 130.

As a preferred embodiment of the present disclosure, the user terminal 120 may be implemented in the form of a smartphone, a smartwatch, a hand-held device, a wearable device, etc. Additionally, applications for using the smart gym system 100 may be installed on the user terminal 120. The user terminal 120 may receive exercise sequence information, etc. from the smart gym server 110. The exercise sequence refers to an exercise plan created considering the user's physical strength and exercise capacity. The exercise sequence includes information, such as a list of exercise equipment to be used by a user, and a target weight and the number of uses of each exercise equipment.

When using at least one piece of the exercise equipment 100A, 100B, 100C, and 100N in the smart gym system 100, the user may use the terminal 120 to communicate with the exercise equipment through tagging, such as NFC or RFID or may use the user's physical characteristics to verify the user's identity. Once the user's identity is verified, the smart gym server 110 may transmit user data to the exercise equipment tagged by the user.

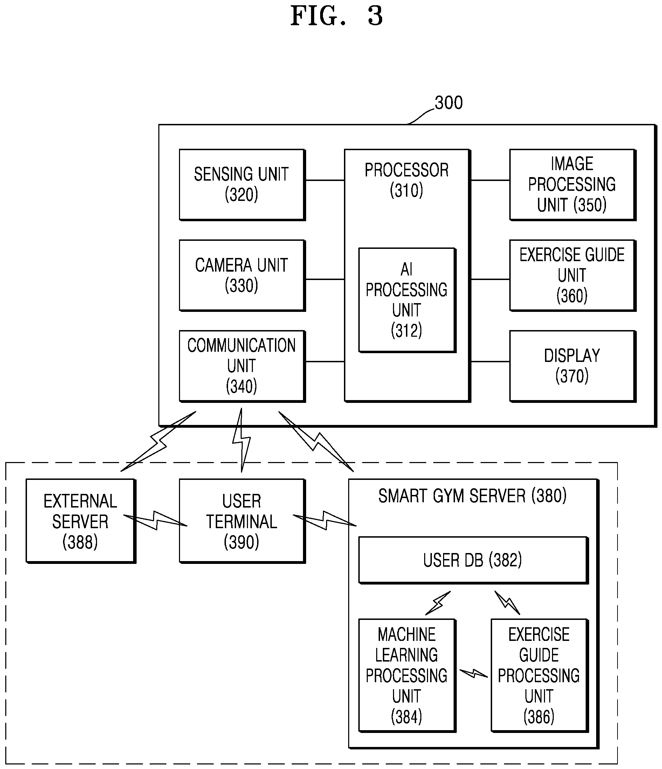

FIG. 3 is an internal configuration diagram of exercise equipment implemented with an AI exercise guide method and a smart gym server in a smart gym system, as a preferred embodiment of the present disclosure.

As a preferred embodiment of the present disclosure, exercise equipment 300 in a smart gym may communicate with a smart gym server 380, a user terminal 390, and an external server 388.

As a preferred embodiment of the present disclosure, the exercise equipment 300 includes a processor 310, a sensing unit 320, a communication unit 340, an exercise guide unit 360, and a display 370. In addition, the exercise equipment 300 may further include a camera unit 330 and an image processing unit 350. The processor 310 may further include an AI processing unit 312, if necessary.

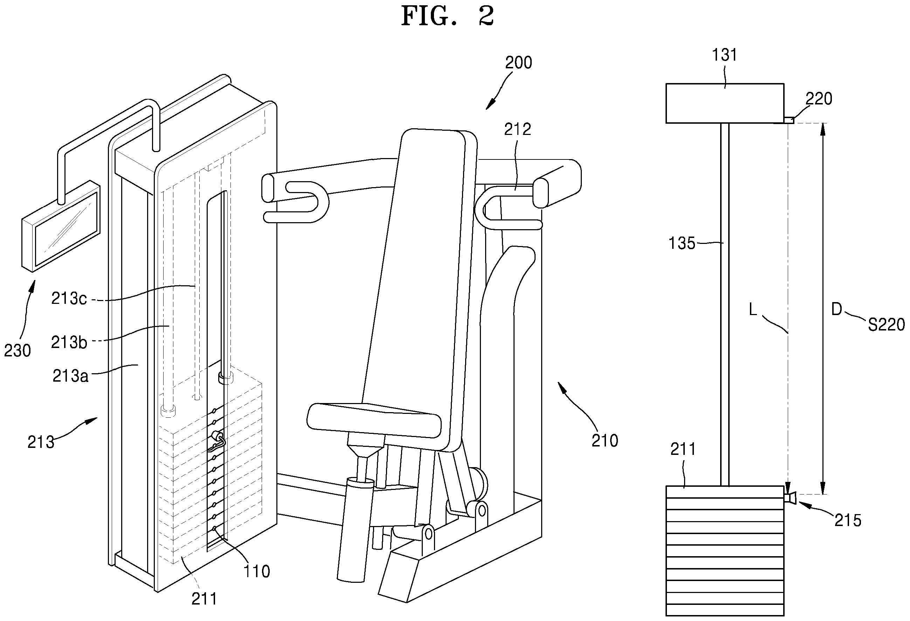

Referring further to FIG. 2, a shoulder press 200 represents an example of the exercise equipment 300 to which an AI exercise guide method is applied, used in a smart gym. The exercise equipment 300 is described with reference to FIGS. 2 and 3.

The sensing unit 220 may be installed on a frame structure 213 of a body 210 of the shoulder press 200. The frame structure 213 includes a base frame 213a, a guide rail 213b, and a connection line 213c. The sensing unit 220 radiates a laser beam toward a pin structure 215, receives the reflected laser beam, and measures the distance D (S220) from the sensing unit 220 to the pin structure 215 in real time or in a preset time unit. The sensing unit 220 may detect at least one of the position, the moving speed, and the moving direction of a weight member 211 selected by the pin structure 215 in real time. In addition, when the user pushes a handle 212 of the shoulder press 200 to move a weight plate, the sensing unit 220 may measure the distance D (S220) to the pin structure 215 inserted into the weight plate and may detect exercise traces based thereon.

The communication unit 340 may receive user input through the display unit 230 or exchange user data with a user DB 382 of the smart gym server 380. The communication unit 340 may also communicate with the external server 388.

The exercise guide unit 360 may provide a user with information, such as a target weight of exercise equipment, a moving speed of exercise equipment, a breathing method when using exercise equipment, and an exercise sequence when using multiple exercise equipment, based on user data received from the user DB 382.

The smart gym server 380 may calculate an estimated muscle strength value of a specific muscle strength based on the user data. In addition, PMWₑₛₜᵢₘₐₜₑ of the exercise equipment to be used by the user is estimated based on the estimated muscle strength value, and an initial target weight of the exercise equipment to be used by the user is automatically set based on the user's exercise purpose and PMWₑₛₜᵢₘₐₜₑ. In a preferred embodiment of the present disclosure, a personal maximum weight (PMW) refers to a muscle strength that an individual can exert against the resistance of a weight with maximum effort. Examples of the PMW include 1RM, 4RM, etc.

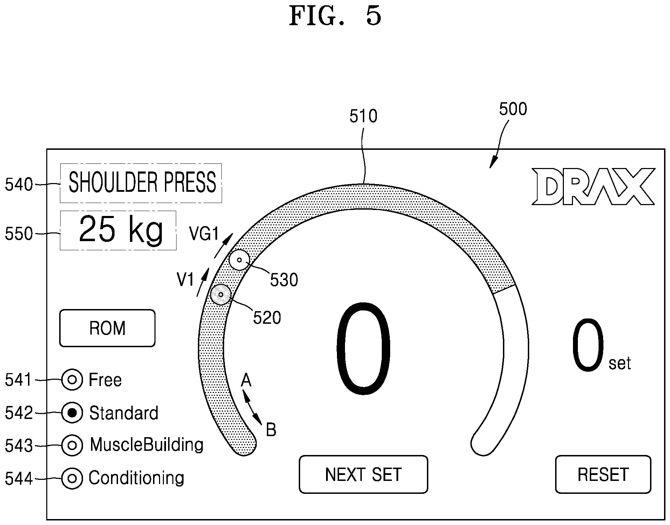

The smart gym server 380 may provide the initial target weight of the exercise equipment to be used by the user to the exercise guide unit 360 of the exercise equipment 300, and the exercise guide unit 360 may display the initial target weight of the exercise equipment to be used by the user on the display 370. FIG. 5 is referred to for an example presenting the initial target weight (550 in FIG. 5) of the exercise equipment to be used by the user.

The camera unit 330 may be built into the exercise equipment 300 or may be capable of wired or wireless communication with the exercise equipment 300, and may capture the user's exercise posture. The image processing unit 350 may analyze the user's exercise posture captured by the camera unit 330 through the processor 310. In addition, the AI processing unit 312 may learn the image processing results of the exercise posture captured when the user exercises according to the target weight of the exercise equipment and transmit the learning results to the smart gym server 380. The AI processing unit 312 may also process or learn an objectification index of exercise equipment used by a user. An example of the objectification index is an exercise record, and the exercise record includes the weight of exercise equipment, the number of repetitions (reps), the number of sets, exercise traces, the movement speed, and the regularity of reps per set, each identified when using the exercise equipment.

The smart gym server 380 includes the user DB 382, a machine learning processing unit 384, and an exercise guide processing unit 386.

The user DB 382 stores and manages user data. The user data includes a user's gender, age, weight, height, BMI, and body fat percentage.

The machine learning processing unit 384 learns and processes the objectification index including the exercise record of the exercise equipment used by the user in the smart gym. The machine learning processing unit 384 may update the PMWₑₛₜᵢₘₐₜₑ to PMW_{individual} based on the objectification index of the exercise equipment used by the user in the smart gym for a certain period. The machine learning processing unit 384 may update the PMW_{individual} to be greater than the PMWₑₛₜᵢₘₐₜₑ when the objectification index is greater than or equal to a first reference value and may update the PMW_{individual} to be less than the PMWₑₛₜᵢₘₐₜₑ when the objectification index is less than or equal to a second reference value.

That is, when it is determined that the user's exercise capacity is greater than the PMWₑₛₜᵢₘₐₜₑ estimated from the user's specific muscle strength based on the objectification index identified based on the user's exercise record, the machine learning processing unit 384 may reset the user's maximum muscle strength value to PMW_{individual} greater than the PMWₑₛₜᵢₘₐₜₑ. On the contrary, when it is determined that the user's exercise capacity is less than the PMWₑₛₜᵢₘₐₜₑ estimated from the user's specific muscle strength based on the objectification index identified based on the user's exercise record, the machine learning processing unit 384 may reset the user's maximum muscle strength value to PMW_{individual} less than the PMWₑₛₜᵢₘₐₜₑ.

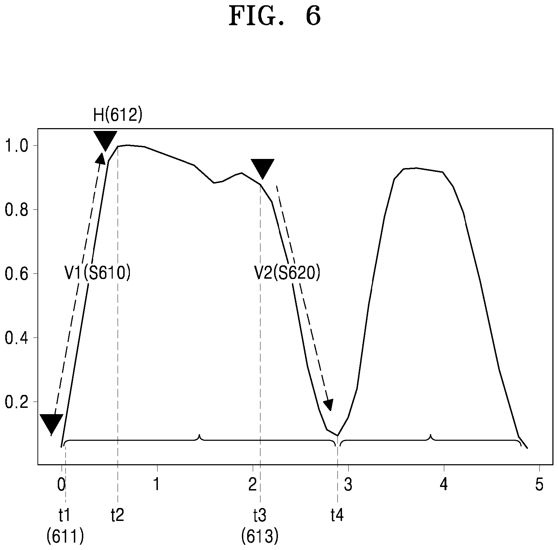

Referring further to FIGS. 5 and 6, the user may move a shoulder press according to a movement speed guide VG1 530 provided by the exercise guide unit 386. The movement speed guide VG1 530 refers to a movement speed for obtaining an effect suitable for the exercise purpose when the user uses the shoulder press. When the user pushes the shoulder press according to the movement of the movement speed guide VG1 530, a measured speed V1 520 at which the user pushes the shoulder press is displayed on a reference line 510. The AI processing unit 312 or the exercise guide processing unit 386 determines that the higher the matching rate between the movement speed guide VG1 530 and the speed V1 520, the more optimized exercise has been performed for the exercise purpose. The sensing unit 320 may determine the exercise traces based on distance information sensed while the user uses the shoulder press. The machine learning processing unit 384 determines that the higher the matching rate between the movement speed guide VG1 530 and the speed V1 520, the more appropriately the user uses the shoulder press according to the exercise program provided by the AI exercise guide device.

The machine learning processing unit 384 may determine the completeness of the exercise traces using at least some of an ascent start point 611, a descent start point 613, an ascending section speed V1 S610, a descending section speed V2 620, an ascending section average speed, a descending section average speed, and a height H 612, each identified from the exercise traces detected while the user was using the shoulder press, and may convert the degree of the completeness of the exercise traces into a number and use the same as an objectification index.

When determining the completeness of the exercise traces, the machine learning processing unit 384 may convert the degree of the completeness thereof into a number using a method of assigning a score of high, medium, low, or 1 to 10 points thereto based on preset criteria and use the same as the objectification index, by comparing preset values with the ascent start point, the descent start point, the ascending section speed, the descending section speed, the ascending section average speed, the descending section average speed, and the height, respectively. It should be noted that this is only an example of the present disclosure and various modifications are possible.

As a preferred embodiment of the present disclosure, when using the reps as an objectification index, the machine learning processing unit 384 may determine the completeness of the reps based on the regularity between the exercise traces of the total number of repetitions constituting one set and may convert the degree of completeness of the reps into a number.

The exercise guide processing unit 386 may also provide an updated target weight of exercise equipment based on the PMW_{individual} received from the machine learning processing unit 384.

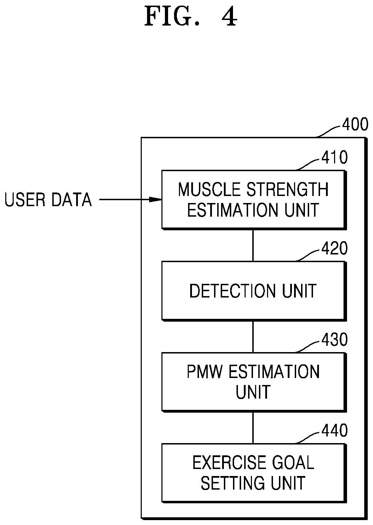

FIG. 4 is an internal configuration diagram of an AI exercise guide device, as a preferred embodiment of the present disclosure.

The AI exercise guide device 400 includes a muscle strength estimation unit 410, a detection unit 420, a PMW estimation unit 430, and an exercise goal setting unit 440.

The strength estimation unit 410 estimates an estimated muscle strength value for the user's specific muscle strength based on the user data. The strength estimation unit 410 uses the user's gender, BMI, body fat percentage, and age information to calculate an estimated muscle strength value for the user's specific muscle strength as in one embodiment of Equation 1. An example of the specific muscle strength may be a grip strength. An estimated muscle strength value = A*gender + B*age + C*body fat percentage +D*BMI + E

In Equation 1, A, B, C, D, and E represent preset values.

The detection unit 420 detects a percentile value which is obtained from the external server (388 in FIG. 3) or detects a percentile value to which the estimated muscle strength value belongs among the preset adult muscle strength percentile values as shown in Tables 1 and 2. Table 1 shows percentile values of relative grip strength for adult men, and Table 2 shows percentile values of relative grip strength for adult women.

**[Table 1]**

| | Age | | | | |
|---|---|---|---|---|---|
| Percentile | 19-29 | 30-39 | 40-49 | 50-59 | 60-69 |
| 90 | **.* | ... | | | **.* |
| ... | | | | | |
| 50 | 57.2 | | 56.1 | **.* | **.* |
| 40 | **.* | 55.9 | **.* | 53.8 | 49.2 |
| ... | | | | | |
| 10 | **.* | ... | | | **.* |

**[Table 2]**

| | Age | | | | |
|---|---|---|---|---|---|
| Percentile | 19-29 | 30-39 | 40-49 | 50-59 | 60-69 |
| 90 | **.* | ... | | | 47.5 |
| ... | | | | | |
| 50 | **.* | 46.3 | 43.8 | **.* | **.* |
| 40 | 40.3 | **.* | **.* | **.* | 34.1 |
| ... | | | | | |
| 10 | 31.7 | ... | | | ** * |

For example, when the estimated muscle strength value for a 32-year-old man is calculated as 55.9 by the muscle strength estimation unit 410, the detection unit 420 detects a percentile value of 40 to which the estimated muscle strength value of 55.9 for a 32-year-old man belongs, among the preset adult muscle strength percentile values as shown in Table 1.

The PMW estimation unit 430 estimates the PMW of the exercise equipment that the user intends to use using the PMW percentile value that matches the percentile value of the estimated muscle strength value detected by the detection unit 420. As a preferred embodiment of the present disclosure, the PMW estimation unit 430 may pre-store the PMW percentile value of the exercise equipment obtained from the population using the exercise equipment or download the same from a smart gym server.

The smart gym server pre-stores a mapping table that stores a PMW percentile value for each exercise equipment, and the PMW percentile value may be updated on a preset time unit by further reflecting the data of users using each exercise equipment in a smart gym. More specifically, the machine learning processing unit 384 of the smart gym server 380 generates a matching table based on PMW data obtained from an initially preset population. The machine learning processing unit 384 may continuously collect PMW data of users who use exercise equipment provided in the smart gym and may update the matching table that provides PMW percentile values generated based on PMW data acquired from the initially preset population in a certain time unit.

Tables 3 to 5 show examples of the mapping table used by the PMW estimation unit 430. For convenience of explanation, Tables 3 to 5 show only portions of PMW10 to PMW90 percentile values, but the listed values are only an embodiment for understanding.

As a preferred embodiment of the present disclosure, an example of the matching table with PMW percentile values of exercise equipment obtained from a population using a chest press by the PMW estimation unit 430 is shown in Table 3.

**[Table 3]**

| PMW percentile | Initial population PMW (kg) | Updated population PMW (kg) |
|---|---|---|
| PMW90 | 102.7 | 107.8 |
| ... | | |
| PMW50 | 67.2 | 67.8 |
| PMW40 | 65.1 | 64.7 |
| ... | | |
| PMW10 | 24.6 | 23.9 |

As a preferred embodiment of the present disclosure, an example of the matching table with PMW percentile values of exercise equipment obtained from a population using a leg extension by the PMW estimation unit 430 is shown in Table 4.

**[Table 4]**

| PMW percentile | Initial population PMW (kg) | Updated population PMW (kg) |
|---|---|---|
| PMW90 | 103.3 | 107.8 |
| ... | ... | ... |
| PMW50 | 67.3 | 69.2 |
| PMW40 | 66.5 | 66.8 |
| ... | ... | ... |
| PMW10 | 24.6 | 24.9 |

As a preferred embodiment of the present disclosure, an example of the matching table with PMW percentile values of exercise equipment obtained from a population using a shoulder press by the PMW estimation unit 430 is shown in Table 5.

**[Table 5]**

| PMW percentile | Initial population PMW (kg) | Updated population PMW (kg) |
|---|---|---|
| PMW90 | 64.5 | 65.5 |
| ... | ... | ... |
| PMW100 | 43.3 | 43.2 |
| PMW40 | 41.2 | 40.2 |
| ... | ... | ... |
| PMW10 | 16.1 | 16.3 |

When a percentile value of 40, to which the estimated muscle strength value of 55.9 for a 32-year-old man belongs, is detected among the preset adult muscle strength percentile values as shown in Table 1, the PMW estimation unit 430 estimates the PMW of the exercise equipment corresponding to the percentile value of 40. Referring to Tables 3 to 5, the PMW estimation unit 430 estimates the PMWₑₛₜᵢₘₐₜₑ of the chest press as 65.1 kg, corresponding to the percentile value of PMW40, based on the initial population PMW (kg). The PMW of the leg extension is estimated to be 66.5kg, which corresponds to the percentile value of PMW40. In addition, the PMW of the shoulder press is estimated to be 41.2kg, which corresponds to the percentile value of PMW40.

As a preferred embodiment of the present disclosure, when additional big data of users using exercise equipment in a smart gym is collected in addition to the PMW data of the initially preset population, the PMW estimation unit 430 may use the updated PMW data, such as updated population PMW (kg) in Tables 3 to 5. In this case, the PMWₑₛₜᵢₘₐₜₑ of the chest press may be updated to 64.7kg, which corresponds to the percentile value of PMW40. The PMWₑₛₜᵢₘₐₜₑ of the leg extension may be updated to 66.8kg, which corresponds to the percentile value of PMW40. Additionally, the PMWₑₛₜᵢₘₐₜₑ of the shoulder press may be updated to 40.2kg, which corresponds to the percentile value of PMW40.

The exercise goal setting unit 440 sets the initial target weight of each exercise equipment based on the PMWₑₛₜᵢₘₐₜₑ of each exercise equipment estimated by the PMW estimation unit 430. The exercise goal setting unit 440 may set an initial target value for each exercise equipment according to the user's exercise goal received through the user interface.

The exercise goal setting unit 440 may set the initial target value of each exercise equipment differently depending on whether the user's exercise goal is free exercise 541, standard exercise 542, muscle building 543, or conditioning 544.

In the case of free exercise 541, the initial target value of the exercise equipment may be set as PMWₑₛₜᵢₘₐₜₑ * P_{Free} (%). In the case of standard exercise 542, the initial target value of the exercise equipment may be set as PMWₑₛₜᵢₘₐₜₑ * P_{standard} (%). In the case of muscle building 543, the initial target value of the exercise equipment may be set as PMWₑₛₜᵢₘₐₜₑ * P_{muscle-building} (%). In the case of conditioning 544, the initial target value of the exercise equipment may be set as PMWₑₛₜᵢₘₐₜₑ * P_{conditioning} (%).

As a preferred embodiment of the present disclosure, P_{muscle-building} (%) may be set to 65% to 85%, and the number of repetitions may be set to 6 times to 12 times. As a preferred embodiment of the present disclosure, when the purpose of exercise is muscle building, the weight P_{muscle-building} (%) to be reflected may use a preset value. In addition, the weight P_{muscle-building} (%) may be set to be readjusted by receiving the objective index feedback, such as the user's exercise traces for the initial target weight suggested to the user and the completeness of the exercise traces for each number of exercises.

When it is assumed that the user selects the standard exercise 542 as the exercise purpose through the user interface provided on the display 500, the exercise goal setting unit 440 may set 25 kg as the initial target weight after reflecting, for example, P_{standard} (%) = 60, when the PMWₑₛₜᵢₘₐₜₑ of the shoulder press is 40.2kg. Since 40.2 kg*60% = 24.12 kg, the closest weight of 25 kg may be set as the initial target weight among the weights supported by the exercise equipment. As a preferred embodiment of the present disclosure, the exercise goal setting unit 440 may set and provide a new target weight based on the PMW_{individual} of the shoulder press.

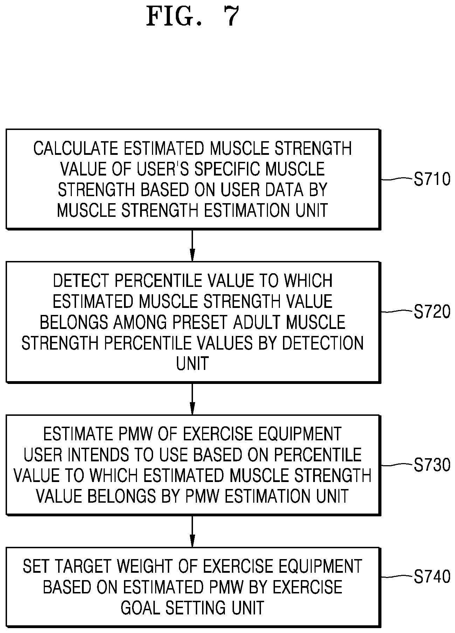

FIG. 7 is a flowchart of an AI exercise guide method, as a preferred embodiment of the present disclosure.

When a user tags exercise equipment he or she wants to use, a smart gym server may transmit user data to the exercise equipment. Alternatively, the user may directly input his/her user data using a user interface supported by a display attached to a terminal or exercise equipment. A muscle strength estimation unit calculates an estimated muscle strength value of a user's specific muscle strength based on user data (S710). The specific muscle strength of the user may be a grip strength.

A detection unit detects a percentile value to which the estimated muscle strength value belongs among preset adult muscle strength percentile values (S720). A PMW estimation unit estimates a PMW of exercise equipment the user intends to use based on the percentile value to which the estimated muscle strength value belongs (S730). The PMW estimation unit may use a PMW percentile value corresponding to the percentile value to which the estimated muscle strength value belongs. For example, when the percentile value to which the estimated muscle strength value belongs is a% (a is a natural number), a PMW of the exercise equipment is estimated using a value obtained by multiplying the PMW percentile value by a%. An exercise goal setting unit sets the target weight of the exercise equipment based on the estimated PMW (S740).

Methods according to an embodiment of the present disclosure may be implemented in the form of program instructions that can be executed through various computer means and may be recorded on a computer-readable medium. The computer-readable medium may include program instructions, data files, data structures, etc., solely or in combination. The program instructions recorded on the computer-readable medium may be those specifically designed and configured for the present disclosure, or may be known and usable by those skilled in the art of computer software.

As described above, the present disclosure has been described using limited embodiments and drawings, but is not limited to the above embodiments, and various modifications and variations can be made from the descriptions by those skilled in the art.
Industrial Applicability
Sequence List Text
PRIOR ART DOCUMENTS
PATENT DOCUMENTS
NON-PATENT DOCUMENTS
EXPLANATION OF REFERENCE NUMERALS DESIGNATING THE MAJOR ELEMENTS OF THE DRAWINGS

## Claims

1. An AI exercise guide device comprising:
a muscle strength estimation unit for estimating an estimated muscle strength value of a user's specific muscle strength based on user data;
a detection unit for detecting a percentile value to which the estimated muscle strength value belongs among preset adult muscle strength percentile values; and
a PMW estimation unit for estimating a PMW of exercise equipment the user intends to use based on the detected percentile value.

2. The AI exercise guide device of claim 1, further comprising an exercise goal setting unit for automatically setting a target weight of exercise equipment the user intends to use based on the estimated PMW thereof.

3. The AI exercise guide device of claim 1, wherein the PMW estimation unit is configured to estimate a PMW corresponding to the estimated muscle strength value by matching a PMW percentile value of exercise equipment obtained from a population using the exercise equipment the user intends to use with the preset adult muscle strength percentile value, using a matching table.

4. The AI exercise guide device of claim 3, wherein the PMW percentile value of the exercise equipment obtained from the population is updated using PMW data of the user who used exercise equipment the user intended to use in at least one smart gym, and in this case, the matching table is updated.

5. The AI exercise guide device of claim 1, wherein the user data comprises at least some of a user's gender, age, weight, height, BMI, and body fat percentage.

6. The AI exercise guide device of claim 1, wherein the specific muscle strength comprises a grip strength.

7. The AI exercise guide device of claim 2, wherein a target weight of exercise equipment that the user intends to use varies depending on the user's exercise purpose received through a user interface.

8. An AI exercise guide method comprising:
calculating, by a muscle strength estimation unit, an estimated muscle strength value obtained by estimating a user's specific muscle strength based on user data;
detecting, by a detection unit, a percentile value to which the estimated muscle strength value belongs among preset adult muscle strength percentile values; and
estimating, by a PMW estimation unit, a PMW of exercise equipment the user intends to use based on a percentile value to which the estimated muscle strength value belongs.

9. The AI exercise guide method of claim 8, further comprising setting, by an exercise goal setting unit, a target weight of exercise equipment that the user intends to use based on the estimated PMW.

10. The AI exercise guide method of claim 9, wherein the target weight is automatically set.

11. The AI exercise guide method of claim 8, wherein the estimating of the PMW comprises estimating a PMW matching the estimated muscle strength value by using a matching table that matches a PMW percentile value of exercise equipment obtained from a population using the exercise equipment that the user intends to use with the preset adult muscle strength percentile value.

12. The AI exercise guide method of claim 8, wherein the user data comprises at least some of a user's gender, age, weight, height, BMI, and body fat percentage.

13. The AI exercise guide method of claim 8, wherein the specific muscle strength comprises a grip strength.

14. The AI exercise guide method of claim 9, wherein a target weight of exercise equipment that the user intends to use is automatically set differently depending on the user's exercise purpose received through a user interface.

15. A computer-readable recording medium, wherein the computer-readable recording medium stores instructions that cause a computing device to perform the following method, the method comprising:
calculating, by a muscle strength estimation unit, an estimated muscle strength value obtained by estimating a user's specific muscle strength based on user data;
detecting, by a detection unit, a percentile value to which the estimated muscle strength value belongs among preset adult muscle strength percentile values;
estimating, by a PMW estimation unit, a PMW of exercise equipment the user intends to use based on a percentile value to which the estimated muscle strength value belongs; and
setting, by an exercise goal setting unit, a target weight of exercise equipment that the user intends to use based on the estimated PMW.
